# EUROPEAN PATENT APPLICATION

(11) **EP 0 911 006 A1**
(43) Date of publication of application: **28.04.1999**
(21) Application number: 97945887.4
(22) Date of filing: 04.12.1997
(51) Int. Cl.: A61F 13/15, A61F 5/451

(54) **REUSABLE DIAPER**

(30) Priority: 04.12.1996 ES 9600258; 04.07.1997 ES 9700149
(71) Applicant: Valladares Pereda, Victoriano, 29014 Malaga (ES); Bautista Moreno, Maria Lucia, 29014 Malaga (ES)
(72) Inventor: Valladares Pereda, Victoriano, 29014 Malaga (ES); Bautista Moreno, Maria Lucia, 29014 Malaga (ES)
(86) International application number: ES9700298
(87) International publication number: WO9824388

(57) **Abstract**

Re-usable diaper which comprises an impervious laminar body (1) which has an inner thin covering. Said diaper is of a suitable configuration to accommodate to the user's crotch and to attach to the user's waist. Said diaper presents a ring-shaped resilient shirring (5) in its central or middle zone, which determines a hollow or cavity (7) which forms a sort of collection bag (8) for fecal excrements. A pad (9) or any other absorbent element of any suitable conventional type is placed in the inside of said cavity (7). Said pad (9) is the element which receives the aforementioned fecal excrements, this pad being exchangeable, thus permitting the diaper to be re-used. Said pad (9) can be optionally refilled with any cereal or grain bran (36). A hole (10) is situated before and next to the ring-shaped shirring (5). The entrance (11) of said hole (10) is resiliently shirred, being destined to permit the exit of the male urinary organ, or the female urine collection device.

## Description

### OBJECT OF THE INVENTION

This descriptive report refers to a reusable diaper, specially conceived for incontinent users, who suffer from urinary and bowel incontinence. From the hygienic point of view this diaper offers optimum services to the user. It can be reused a considerable number of times, this meaning or involving beneficial repercussions from the economic and environmental point of view, without the detriment of its services from the sanitary point of view.

This invention presents optionally the possibility of incorporating a non-cellulosic material incorporated into the inside of the bag which carries it, thus redounding to the obtainment of a completely ecologic diaper which avoids the deforestation of the planet. This invention presents optimum services from the hygienic point of view not only for the user but for those who look after incontinent users.

### FIELD OF THE INVENTION

This invention relates to the field of diaper and sanitary towel for human use industry.

### BACKGROUND OF THE INVENTION

At present due to the frequent incontinence cases in adults and children, diapers similar to those used for children are used for every case,though obviously diapers for adults are bigger and preferably the two leg-openings are provided with elastic means to fit the thighs thus assuring a complete stagnancy.

These diapers due to their absorbent nature and their considerable size have a large amount of cellulose and polymers, with the following and negative consequences from the economic and ecologic point of view. These problems are accentuated by the fact that if you want to keep the user in appropriate hygienic conditions, it is necessary to change the diaper a considerable number of times a day using a new diaper after every change.

At the present time and as a consequence of the longevity of human beings, an increase in the use of diapers for urinary or bowel incontinence has been proved. These incontinence problems are produced by the longevity of human beings and are multiplied due to the existence of a lot of patients with lengthy illness derived from traffic accidents.

Diapers for adult human beings and for children have a region which receives faeces or urine. This region is constituted by a pad implemented with different elements and different coatings. This region presents a core formed by cellulose fibres.

In most cases diapers require the use of a supplementary body wich, due to its cellulose nature affixed to the supplementary plastic body, in most cases are removed after having been used, being incapacited to be reused, due to their own configuration characteristics.

In other cases diapers are implemented with an outer cover made of plastic material or similar, destined to retain the sanitary towel or the diaper itself which, due to its characteristics, generates in numerous occasions the need for removing it and laundering it, in order to keep the user in appropiate hygienic conditions.

With the object of solving this problem, the applicant knows the existence of diapers which present a hole in the region corresponding to the user's genital organs. If the user belongs to the male sex, his urinary organ can emerge through said hole, in order to conduct the urine to a collection bag. In this way moisture derived from the urinary discharge will not force to change the diaper.

The user knows the existence of this sort of diapers implemented with an urine collection device which fits to the outer vaginal surface of a woman. The end of the device emerges partially to the outside. This device is funnel-shaped and presents a channelling to which a drainage system is connected. This drainage system will conduct the urine to a collection bag or receptacle.

U.S.Patent No. 4,200,102 discloses a diaper comprising an air-filled kidney-shaped body preferably made of nylon, non-rigid vinyl plastic or the like which has a concave cavity. This concave cavity is placed longitudinally and has a container for excrements which is positioned in the lower part of the user's body and has an inlet for which it adapts to contact the rectal area and has a tubular outlet. Said body is adapted to the crotch region of the user and presents an urine container and is fastened to the user's waist by elastic straps. Said users are preferably babies or adults after major surgery. The body of the diaper can be used constantly , though it has to be cleansed regularly with the objet of keeping the user clean and aseptic.

Obviously this invention can be used sporadically but it can't be used for a prolonged period of time.

U.S.Patent No.4,886,508 discloses a ladies external catheter assembly. It is shaped or configurated like a resiliently deformable elongated body which presents a thin wall next to a cavity or hollow. The inside of said cavity contains a resiliently deformable mesh or screen which tends to return the body to its original shape thus permitting full flow passage of urine to the discharge outlet without significant resistance to flow. This invention has nothing to do with a diaper to be used by the incontinent or handicapped individuals.

U.S.Patent No.,4968,312 discloses a disposable fecal compartmenting diaper comprising a liquid impervious back sheet, an absorbent padding that is elastized, a liner of non-absorbent soft material, following the contour of the wearer's body, and attaching means, compelling to use an urine or fecal excrement receiver diaper.

This invention can't be used in a conventional way, due to its own structure nature and materials employed which compel to use cellulose as a constitutive element of the direct application diaper.

However the applicant doesn't have knowledge at present of the existence of a re-usable diaper which presents a fecal matter receiver structure in whose core cellulose is not used as a receiver material.

Obviously the support body (which is constitutive of the outer attachment area of the diaper) and the diaper itself will have common characteristics in order to facilitate the user's urination and to permit the attachment of the body of the diaper which can be re-used due to its characteristics.

### DESCRIPTION OF THE INVENTION

The re-useble diaper proposed by this invention has been designed to solve the aforementioned problems. Its re-usable characteris due to its structuration which is essentially different to the ones known at present.

The preconized diaper, as any other diaper, has a basic element: a laminar impervious body which is provided with a cellulose or any other hypoallergic material covering in its inner face. Said covering has a minimal thickness because the cellulose or intervening material is not for absorbing but for insulating the plastic material constitutive of the impervious body from the wearer's skin. This diaper incorporates, as its main characteristic, a ring-shaped resilient shirring which is placed in the central region of this diaper. Said resilient shirring faces up to the perineal and anal zones of the wearer. Besides, said resilient shirring forms or delimit a cavity or hollow like a fecal excrement collection bag which is properly sized. Said collection bag is properly isolated from the rest of the diaper due to the pressure that said resilient shirring exerts against the user's body.

This resilient shirting can be substituted by any other mean which delimits a cavity whose function is to form a receptacle. This receptacle is for collecting the individual's fecal excrements. Said receptacle can be a non-detachable part of the diaper or an absorbent exchangeable insert attached to this diaper in its anal region.

The ring-shaped resilient shirring can be ovoid-shaped, elliptic-shaped, ring-shaped, or can have any other suitable shape, though following a closed contour in its inside.

According to another characteristic of this invention, this collecting bag of the laminar body is destined to receive a pad or any other absorbent insert in its inside. Said pad or absorbent insert is properly stabilized by a tab which fits to the resilient shirring of the fecal excrement collection bag. Said pad receives the aforementioned fecal excrements and can be removed, thus keeping the diaper clean, said diaper being re-usable.

According to another characteristic of this invention, there is a hole or orifice situated before and adjacent to said ring-shaped resilient shirring . This orifice is resiliently shirred and is destined to fit tight to the male genital organs , or to a female urine collection device. The urinary system of the user is connected to an urine collection bag, said collection bag being of any conventional type.

This orifice can have any possible or imaginable shape: it can be round-shaped, oval-shaped, square-shaped, rectangular-shaped, elliptical-shaped, etc.

As it has been said before, the diaper itself has a ring-shaped resilient shirring which delimits a cavity in which an absorbent insert is placed (or inserted ). However the diaper can be made flat, or can delimit a small cavity, then said exchangeable absorbent insert defining the exact aspect for the reception of the individual's fecal excrements.

Finally, according to another characteristic of this invention, said laminar body includes a properly shaped pocket in one of its side zones. After having accommodated the diaper to the individual's body, the entrance of said pocket is in its upper part. Said pocket is destined to receive said urine collection bag and, consequently, to keep it properly accommodated to the user.

According to the described structure , not only a substantial amount of cellulose is saved - specially due to its re-usable character - but its volume is substantially reduced, too, with regard to a conventional diaper, thus facilitating its storage and improving use conditions.

It must be explained or clarified that this diaper can be capriciously shaped, said capricious shape being similar to an undergarment which can be like a pair of shorts (having trouser legs), or like panties.

It is a further object of this invention to provide a re-usable diaper which is constituted by the use of an outer support body. Said outer support body is of a rectangular ground plan configuration. The central or middle part of its side edges are cut and are round-arch-shaped, or half-circle shaped. Said round arch or half-circle is resiliently shirred in its central or middle zone.

The middle or central part of the forward and rear edges of said outer support body are resiliently shirred. Said outer support body has retention and attachment means (positioned in its upper or inner surface) which attach to retention and attachment means positioned in its lower or outer surface. Said attachment means and the aforementioned resiliently shirred edges collaborate or work together in order to fit the outer support body to the wearer's body. To be exact, it fits to the lower parts of the user's abdomen, thus covering genital and excretory organs. Said outer support body is made of a non-cellulosic material, as, for example, a transpirable polypropylene or the like, recycled cotton or natural fibre viscose.

There is a reception zone in the central or middle region of one of its surfaces (or faces). Said reception zone projects almost vertically and has a perimeter which is resiliently shirred almost in its entirety, having four holes or orifices which are harmoniously situated in its side walls. Said holes or orifices are destined to permit the passage of the fecal excrement collection bag straps. Said straps permit a proper attachment or fastening of the collection bag to the user.

A suitable zone of the support has been provided with a hole for the exit of the female urine collection device; or the male genital organs (if the user is a man).

The aforementioned absorbent pad or exchangeable disposable insert is attached or fastened to the user with the help of straps. Said insert is stabilized in the middle region of the outer support body. Said insert is made of two layers. The outer or lower layer is configurated like an impervious plastic material thin covering. The upper or inner layer faces directly to the user's skin, said inner layer being made of cotton or polypropylene.

Between both layers or thin coverings there is an absorbent element which is a mixture made of oat bran, or any other cereal or grain bran, or another appropriate product to which polymer is added. Said absorbent mixture contains 1% of polymer. The aforementioned insert is divided into parallel aligned similar bags by means of sealing ,which join the inner or upper layer to the outer or lower layer. Said parallel aligned bags contain the absorbent mixture.

Optionally said absorbent mixture can be made of cellulose to which polymer is added.

The upper layer of the aforementioned exchangeable disposable insert is made of polypropylene or cotton. Said upper layer can incorporate, or can be provided with, a suitable amount of protective and healing oil which is specially appropriated for bed sores, thus protecting the user's skin.

### DESCRIPTION OF THE DRAWINGS

To complement this description, and with the object of helping to a better understanding of the characteristics of this invention, this descriptive report is accompanied by the following figures or drawings, with the understanding that the drawings are illustrative only. Said drawings are not limiting or restrictive.
Figure 1 is a perspective view illustrating the pieces of a re-usable diaper for adults, which is made according to the object of this invention.
Figure 2 is a cross-sectional view of the laminar body which is constitutive of the diaper. The structure of its thickness can be seen.
Figure 3 is a cross-sectional view of the entrance of the fecal excrement collection bag.
Figure 4 is a side view of the same diaper of the previous figures, properly attached to the user's body.
Figure 5 is a ventral-dorsal cross-sectional view of the same diaper of the previous figure, adapted to a male user.

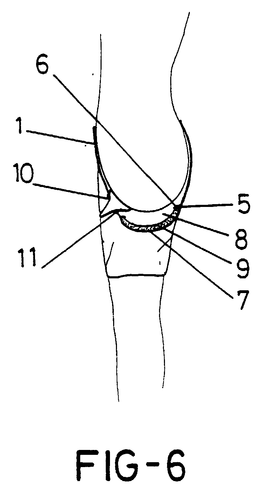

Figure 6 is a ventral-dorsal cross-sectional view of the same diaper of the figure 5, though it is adapted to a female user who is wearing a female urine collection device.

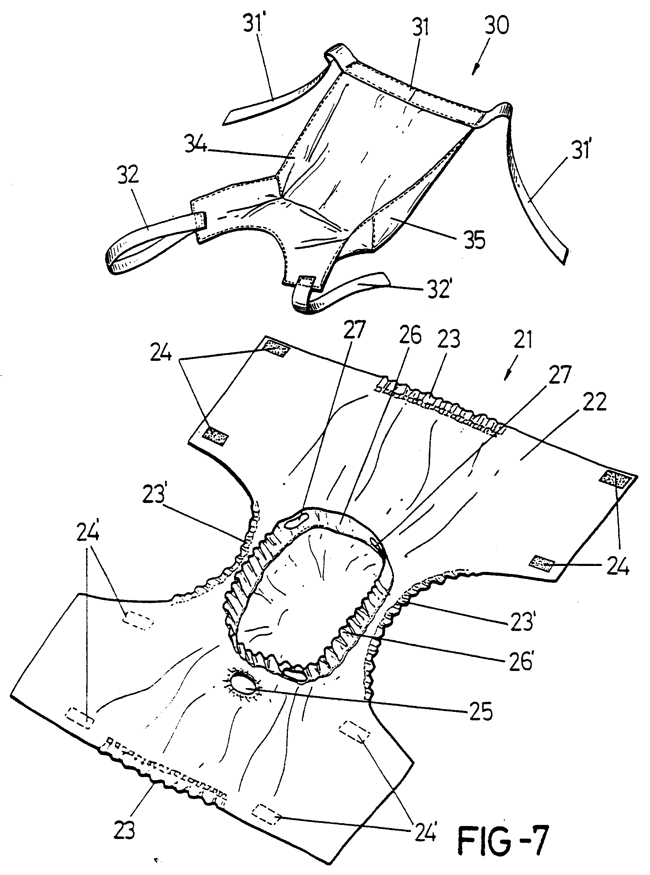

Figure 7 is a perspective view of the outer support body and the absorbent pad (or disposable exchangeable insert), their joint application configurating the object of the invention in a second embodiment.

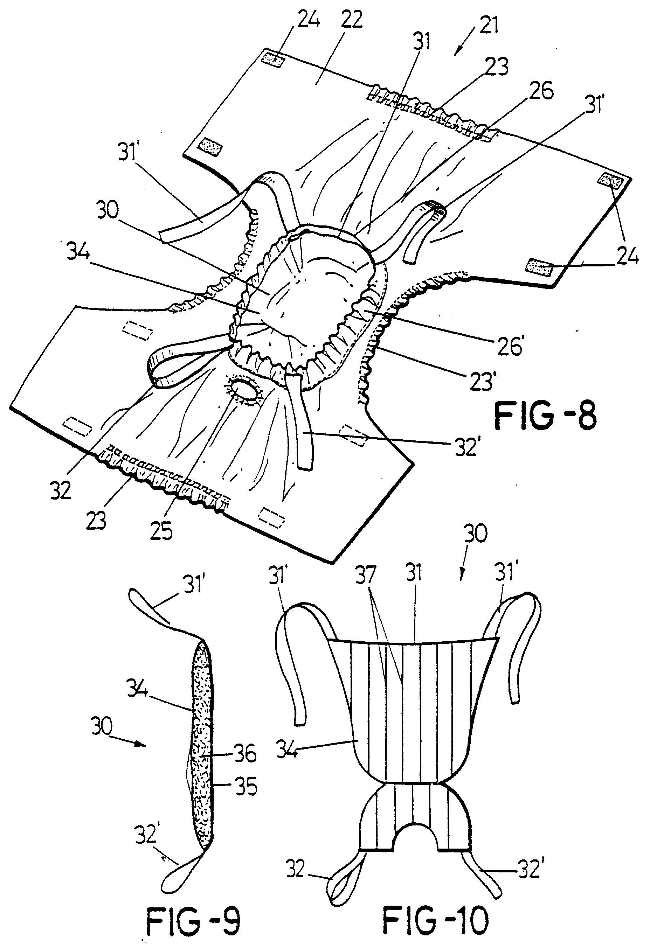

Figure 8 is a perspective view of the objects depicted in the figure 7, properly fixed to each other.
Figure 9 is a cross-sectional view of the ground plant of the absorbent pad or insert.
Figure 10 is a side view of the object depicted in the figure 9.

### DESCRIPTION OF THE PREFERRED EMBODIMENTS.

First of all it must be explained that the following description corresponds to a preferred embodiment of the invention in which a diaper similar to a pair of shorts is shown. Said diaper can be underpant-shaped or panty-shaped.

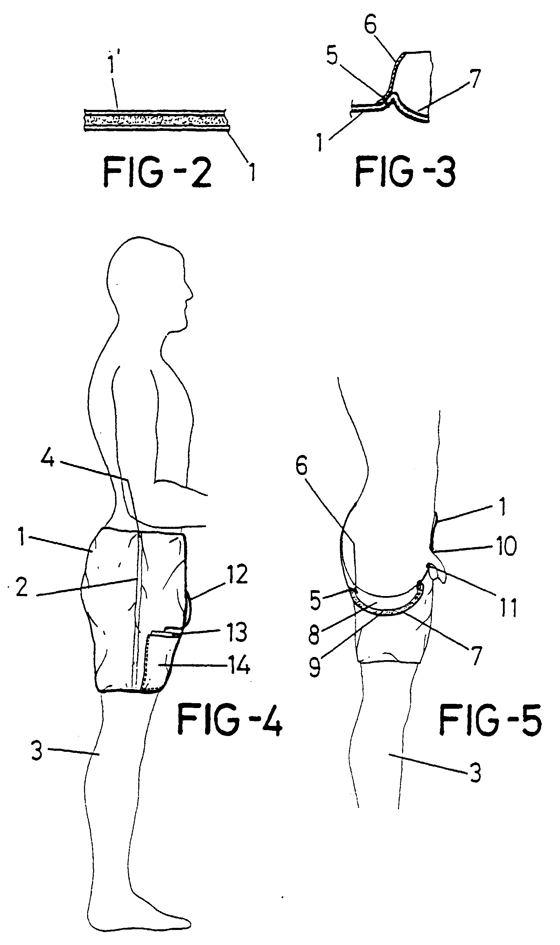

As it can be seen in the attached drawings, the re-usable diaper which is preconized is structured starting from a laminar body (1), materialized in an inmpervious sheet, whose thickness corresponds to the figure 2, provided in its inner side with a cellulose covering, or any other hypoallergic and non-absorbent thin covering. Said laminar body (1) comprises two deep side cuts or notches (2) which delimit the leg-openings for the user's legs (3), as it can be seen in the figure 4, to whose body it is attached by means of adhering its free edges or borders.

Said laminar body (1) presents a ring-shaped resilient shirring (5) to whose seam a flange (6) is fixed. Said resilient shirting (5) and flange (6) are positioned in the middle or central region of the laminar body (1), thus defining a hollow or cavity (7). Said cavity (7) defines a sort of collection bag (8) for fecal bowel excrements. The entrance of said collection bag is constricted by the flange (6), as it can be seen in the figure 5, where it can be seen, too, that said collection bag (8) faces up to the user's perineal zone and anal orifice when it is worn.

Said flange (6) constrictes the entrance of the collection bag (8). A pad or insert (9) is properly stabilized in the inside of said collection bag (8) by the aforementioned flange (6). Said pad or insert is properly sized and configurated to receive said fecal excrements.

In this case, in order to achieve a perfect reception of the excrements, said absorbent pad or insert can have a tab which attaches to the flange (6) in its outer part, thus avoiding soiling the diaper, consequently said diaper being re-usable.

Said resilient shirring can be substituted by attachment means, said means being detachable to allow the removal of the pad or insert, thus making its manufacturing cheaper.

Said laminar body has a small orifice (10) which is situated before said resilient shirring (5). Said orifice (10) is resiliently shirred by an elastic element (11) in such a way that said orifice (10) tends to constrict the male genital organs which emerge from the diaper, as it can be seen in the aforementioned figure 5; or to constrict a female urine collection device, if the user is a woman. This orifice (10) may be in the shape of a circle, an oval, an square, a rectangle, an ellipse, etc.

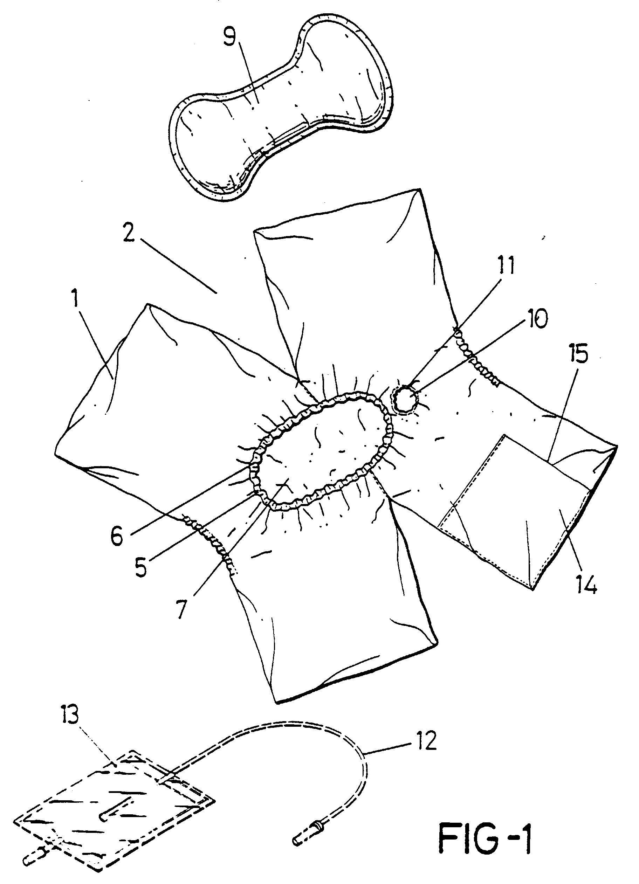

In any case, the patient's urinary system will be connected to a drainage conduit (12). The downstream end of the drainage conduit (12) will communicate with a suitable collection bag (13) for the storage of urine. Said urine collection bag is conventional and known, being drawn in broken lines in the figure 1. Said drainage conduit (12) is connected to the female urine collection device (if the user is a woman).

To facilitate the carriage of the urine collection bag (13), the outer surface of the laminar body is provided with a pocket (14), whose entrance (15) is on the same level as the user's groin is. Said urine collection bag (13) is easily placed into said pocket (14), as can be observed in the figure 4.

In this way, the diaper is light and comfortable, and is not soiled by urinary excretions, because the urinary excretions are channelled directly into the urine collection bag (13). Fecal excrements are received by the pad (9), which is placed into the cavity or hollow (7). Said cavity or hollow (7) is defined within the diaper itself so that said pad (9) is perfectly stabilized in its functional place and can be easily substituted when necessary, the rest of the diaper being re-usable, in optimal hygienic conditions for the user.

Another advantage of this diaper is its compact storage. Said diaper is made of a thin laminar element, thus permitting the compact storage of numerous diapers, contrary to what nowadays happens.

In accordance with a second embodiment this re-usable diaper can be constituted from an outer support body (21), which is made of a non-cellulosic material, specifically polypropylene or the like, or recycled cotton, or natural fibre viscose (22). The middle or central part of its forward and rear edges are resiliently shirred (23). Said outer support body has side edges which are cut and round arch-shaped, or half-circle- shaped. Said round arch or half-circle is resiliently shirred in its central or middle zone (23'). Said outer support body has attachment means (24) in its upper face. Said attachment means (24) attach to attachment means (24') which are prefixed to the lower face. Said outer support body has a hole (25) which is positioned in the zone corresponding to the user's urinary system . This hole is for the exit of the male genital organs (if the user is a man ) or for the exit of the female urine collection device (if the user is a woman). The urinary system of the user is connected to a supple channelling which is destined to conduct the urine to a collection bag. Said outer support body is of a rectangular ground plan configuration. Its inner or upper face (22) has a central or middle region to which a strap is attached. Said strap projects semi-vertically and presents a non-supple zone (26) which has two holes (27). The rest of this central or middle zone presents a resiliently shirred configuration (26') which is provided with two holes (27), thus forming a cavity. An absorbent insert (30) will attach to said cavity.

The aforementioned disposable exchangeable insert (30) has a forward edge or border and a rear edge or border. Said rear edge presents a strap (31) which is made of a supple material as, for example, cotton. Said strap has two supple extensions (31') which will pass through two aligned holes or orifices (27), said holes (27) being positioned in the non-shirred zone (26). The forward edge of said disposable insert (30) presents two supple extensions (32) and (32') which will pass through the other two holes (27) existing in the resiliently shirred zone, thus forming fastening means. Attachment means (24) and (24') accommodate the outer support body (21) to the user's body with the help of elastic means (23) and (23').

Said disposable insert (30) presents a lower or outer face which is destined to be fixed to the inner or upper face of the outer support body (21). Said lower face presents an impervious plastic thin covering (35). This disposable insert (30) has an upper or inner face (34) which presents a sheet, made of polypropylene or cotton, which is permeable in its entirety, thus forming a bag. Said bag contains an absorbent inner element which is a mixture made of oat bran, or any other cereal or grain bran. The body of this insert (30) is divided into longitudinal bags. Said longitudinal bags are delimited by sealing lines.

The aforementioned mixture (36) made of oat bran, or any other cereal or grain bran can be substituted by an appropriate product which will be added 1% of polymer. Said appropriate product can be cellulose optionally, said cellulose being added 1% of polymer.

The upper or inner face (34) of said disposable insert (30) can be provided with a protective and healing oil to avoid or prevent the possible appearance of bed sores in the user's skin.

To sum up, the second embodiment of this invention incorporates, as an outer support body, (21) a polipropylene or similar sheet (22) which can be substituted by recycled cotton or natural fibre viscose, capable of being properly laundered when there is a need for being disinfected.

Due to the characteristics of the disposable insert (30) and due to the nature of the core or mixture (36) which receives the faeces, there is available an element capable of receiving the body fluids. That element is the absorbent mixture, which is made of natural products. This mixture doesn't affect or influence negatively to the destruction of the environment, which is mainly due to the excessive use of cellulose. Besides, the upper face (34) of the disposable insert (30) is provided with a protective and healing oil. Due to the aforementioned characteristics, there is avoidable a new diaper, provided with ( unknown until today ) characteristics.

The outer support body has two side edges whose central or middle zone is cut round arch-shaped. The central or middle zone of said round arch is resiliently shirred (23'). Said resilient shirrings (23') help to accommodate the diaper to the user's anatomy.

## Claims

1. Re-usable diaper similar to those constituted by a laminar body which accommodates to the user's crotch and attaches to the wearer's waist by adhesive means, characterized in that said diaper doesn't contain cellulose, polymer, or any other absorbent material, and defines a ring-shaped resilient shirring (5) or cavity in the middle or central region of the laminar body (1). Said elastic shirring (5) determines a cavity or collection bag (7) which faces up to the user's perineal and anal zone when it's worn. Before said ring-shaped resilient sherring (5) there is a hole (10) whose diameter is small and whose perimeter (11) has elastic means to constrict. It is properly positioned to engage to the male genital organs. Said hole is an exit for said male genital organs, or for a female urine collection device. Fecal excrements are received by the aforementioned cavity or collection bag (7). Urinary excrements are collected or stored by an urine collection bag (13) which can be of any conventional type. Said urine collection bag is properly connected to the urinary system of the male user or to the female urine collection device, thus giving a re-usable character to the diaper.

2. Reusable diaper, according to claim 1,characterized in that the laminar body (1) constitutive of the diaper comprises an impervious thin sheet, preferably made of plastic, to whose inner face a cellulosic or any other hypoallergic material thin sheet (1') is fixed. Said thin sheet (1') contacts with the user's skin.

3. Re-usable diaper, according to any preceding claim, characterized in that the elastic seam which determines the ring-shaped resilient shirring (5) constitutes the attachment mean for a complementary flange (6) which constrictes the entrance of the collection bag (8) which is defined by the aforementioned cavity (7). The aforementioned complementary flange (6) stabilizes a pad (9) or any absorbent element of any conventional type in the inside of said collection bag (8). Said pad or absorbent element (9) receives the fecal excrements and is exchangeable.

4. Re-usable diaper, according to any preceding claim, characterized in that said laminar body (1) incorporates a pocket (14) in its outer face. The entrance (15) of said pocket (14) is in its upper part and is destined to receive or contain the urine collection bag (13).

5. Re-usable diaper, according to claim 4, characterized in that the pad or absorbent element can include or comprise a perimetral tab which attaches to the complementary flange (6) in its outer part, thus avoiding soiling the diaper with faeces.

6. Re-usable diaper, according to any preceding claim, characterized in that said pad or absorbent element can be attached to the diaper by adhesive means or by any other suitable mean. The edges or borders of said absorbent element define a cavity which retains the fecal excrements in its inside.

7. Re-usable diaper, according to any preceding claim, characterized in that said diaper may adopt the shape of a pair of shorts, or may adopt the shape of panties.

8. Re-usable, according to any preceding claim, characterized in that in a second embodiment, the outer support body (21) is made of a polypropylene or the like sheet (22). Said sheet (22) is transpirable and can be substituted optionally by recycled cotton or natural fibre viscose. The central or middle zone of its side edges are cut, and are round arch-shaped. Said round arch is resiliently shirred in its central or middle zone (23'). The reception zone of the outer support body has a non-supple laminar zone (26), provided with two holes (27) in its theoretical vertices. The rest of the laminar zone of the outer support body is a resilient shirring (26') in whose theoretical vertices there are two holes (27).

9. Re-usable diaper, according to claim 8, characterized in that said disposable exchangeable insert (30) contains an outer or lower sheet (35), made of an impervious plastic thin covering, to which an upper sheet (34) is fixed perimetrally and by sealing longitudinal lines. Said upper sheet (34) is made of polypropylene or cotton. The rear edge of the absorbent pad (30) presents a cotton strap (31) which has two side extensions (31'). The forward edge of the absorbent pad (30) presents two laminar extensions (32) and (32') respectively.

10. Re-usable diaper, according to claims 8 and 9, characterized in that said impervious lower sheet (35) and permeable upper sheet (34) are joined by sealing means thus forming parallel aligned longitudinal bags which contain a mixture (36) of oat bran, or any other cereal or grain bran, and 1% of polymer.

11. Re-usable diaper, according to claim 10, characterized in that the absorbent mixture optionally made of any cereal or grain bran can be substituted by cellulose, said cellulose being added 1% of polymer.

12. Re-usable diaper, according to claims 8, 9, 10 and 11, characterized in that the disposable exchangeable insert (30) has un upper sheet (34 ) which is adjacent to the user's body, and which is (optionally) provided with a healing protective oil, thus preventing the appearance of bed sores.
